# FASCICULE DE BREVET EUROPEEN

(11) **EP 2 092 037 B1**
(45) Date de publication et mention de la délivrance du brevet: **25.07.2018**
(21) Numéro de dépôt: 07871949.9
(22) Date de dépôt: 17.12.2007
(51) Int. Cl.: C09K 5/04

(54) **COMPOSITIONS UTILISABLES COMME FLUIDE FRIGORIGENE**
KÜHLMITTEL
COMPOSITIONS THAT CAN BE USED AS REFRIGERANTS

(30) Priorité: 19.12.2006 FR 0655613
(43) Date de publication de la demande: 26.08.2009
(60) Demande divisionnaire: 10171556.3
(73) Titulaire: ARKEMA FRANCE, 92700 Colombes (FR)
(72) Inventeur: RACHED, Wissam, F-69007 Lyon (FR)
(74) Mandataire: Chahine, Audrey Claire
(86) Numéro de dépôt international: PCT/FR2007/052533
(87) Numéro de publication internationale: WO 2008/084161

(56) Documents cités:
- EP-A- 1 491 608
- US-A- 3 996 153
- US-A- 4 448 031
- US-A- 5 716 549
- US-A- 5 826 436
- US-A- 6 073 454
- US-A1- 2003 042 463
- US-A1- 2004 084 652
- US-A1- 2004 099 838
- US-A1- 2006 043 331
- US-B1- 6 261 473

## Description

La présente invention concerne des compositions aptes à être utilisées comme fluides frigorigènes. Elle a plus particulièrement pour objet des compositions, dont la contribution à l'effet de serre est très faible, utilisables dans la réfrigération et la production d'air conditionné.

Les problèmes posés par les substances appauvrissant la couche d'ozone atmosphérique ont été traités à Montréal où a été signé le protocole imposant une réduction de la production et de l'utilisation des chlorofluorocarbures (CFC). Ce protocole a fait l'objet d'amendements qui ont imposé l'abandon des CFC et étendu la réglementation à d'autres produits, dont les hydrochlorofluorocarbones (HCFC).

L'industrie de la réfrigération et de la production d'air conditionné a beaucoup investi dans la substitution de ces fluides frigorigènes et c'est ainsi que les hydrofluorocarbures (HFC) ont été commercialisés.

Dans l'industrie automobile, les systèmes de climatisation des véhicules commercialisés dans de nombreux pays sont passés d'un fluide frigorigène au chlorofluorocarbure (CFC-12) à celui de l'hydrofluorocarbure (1,1,1,2 tetrafluoroéthane : HFC-134a), moins nocif pour la couche d'ozone. Cependant, au regard des objectifs fixés par le protocole de Kyoto, le HFC-134a (GWP = 1300) est considéré comme ayant un pouvoir de réchauffement élevé. La contribution à l'effet de serre d'un fluide est quantifiée par un critère, le GWP (Global Warming Potentials) qui résume le pouvoir de réchauffement en prenant une valeur de référence de 1 pour le dioxyde de carbone.

La majorité des installations frigorifiques fonctionnent sur le principe du cycle à compression de vapeur. Suivant ce principe, un fluide frigorifique est évaporé à basse pression, en prenant de la chaleur dans un premier milieu environnant. La vapeur ainsi formée est alors compressée au moyen d'un compresseur et passe ensuite dans un condenseur dans lequel elle est transformée à l'état liquide en donnant lieu à un dégagement de chaleur dans une deuxième zone environnante. Le liquide ainsi condensé circule ensuite dans un détendeur à la sortie duquel il se transforme en un mélange diphasique de liquide et de vapeur, lequel est enfin introduit dans l'évaporateur où le liquide est de nouveau évaporé à basse pression, ce qui complète le cycle. Dans un cycle supercritique, il n'y a pas de condensation et le condenseur est nommé refroidisseur.

La régulation du détendeur permet de contrôler la surchauffe à l'entrée du compresseur et d'optimiser le fonctionnement de l'installation.

Le rendement d'un système à compression dépend des composants, de l'architecture du système, des conditions de fonctionnement et du fluide. Les fluides utilisés peuvent être des corps purs ou bien des mélanges azéotropes ou zéotropes. Le glissement de température d'un fluide (glide) est défini comme la différence de température entre le point de bulle et le point de rosée à pression constante. Dans les systèmes à compression avec un cycle supercritique, le glissement de température est considéré comme la différence de température entre l'entrée et la sortie du refroidisseur.

Dans un cycle à compression théorique les changements de phase (condensation / évaporation) sont à pressions constantes. Avec les fluides monocomposants et les mélanges azéotropes, la condensation et l'évaporation sont à températures constantes. Le glissement de température est alors nul.

Le fonctionnement des climatiseurs, des machines frigorifiques et des pompes à chaleur repose sur les mêmes principes.

Le dioxyde de carbone étant non-toxique, ininflammable et ayant un très faible GWP, a été proposé comme réfrigérant des systèmes de climatisation en remplacement du HFC-134a. Toutefois, l'emploi du dioxyde de carbone présente plusieurs inconvénients, notamment liés à la pression très élevée de sa mise en oeuvre en tant que fluide frigorigène dans les appareils et technologies existantes.

L'utilisation du dioxyde de carbone seul dans un cycle supercritique ou en combinaison avec d'autres composés, tels que les HFC, dans un système frigorifique ou une pompe à chaleur usuelle peut conduire donc à des baisses d'efficacités énergétiques inacceptables si le milieu environnant (air, eau ou eau glycolée) ne présente pas de glissement de température aussi grand que le fluide utilisé. La conséquence est que l'écart entre la température moyenne du
changement de phase d'un mélange qui comprend du dioxyde de carbone et la température moyenne du milieu environnant s'accroît, entraînant alors un accroissement de l'écart entre les pressions d'évaporation et de refroidissement, ce qui a pour conséquence directe de diminuer l'efficacité énergétique du système frigorifique ou de la pompe à chaleur. Cette efficacité énergétique diminue aussi suite à la dégradation de l'efficacité des échangeurs à cause de la variation de l'écart des températures entre le fluide frigorigène et le milieu environnant à travers l'échangeur.

US 6,261,473 décrit un fluide de travail comprenant le 1,1-difluoroéthane, le 1,1,2,2-tétrafluoroéthane, du dioxyde de carbone et un alkyl mercaptan de formule CₙH₂ₙ₊₁SH avec n allant de 1 à 4. US 4,448,031 décrit une composition comprenant de la diéthanolamine et du dioxyde de carbone.

Le document US 6073454 divulgue l'utilisation d'un co-fluide en combinaison avec le dioxyde de carbone comme réfrigérant pour réduire la pression de mise en oeuvre. Toutefois, les performances en réfrigération sont insuffisantes.

Il a maintenant été trouvé des compositions, aptes à être utilisées comme fluides frigorigènes, et ayant un faible GWP. Le GWP des compositions selon la présente invention est de préférence au plus égal à 150.

Il est décrit des compositions comprennant au moins un composé (A) choisi dans le groupe constitué des hydrofluorocarbures, des (hydro)fluorooléfines, (hydro)fluoroiodocarbures et des hydrocarbures, au moins un composé (B) choisi dans le groupe constitué des gaz rares, de l'azote, du dioxyde d'azote, du sulfure d'hydrogène et du dioxyde de carbone et au moins un composé organique fonctionnalisé (C) ayant une température d'ébullition à pression atmosphérique supérieure à 60°C.

Il est décrit que le composé organique fonctionnalisé (C) a une température de fusion à pression atmosphérique inférieure à -20°C et/ou un point éclair > 50°C. Il est décrit que le composé organique fonctionnalisé (C) ea avantageusement une température de fusion à pression atmosphérique inférieure à -20°C et/ou un point éclair > 70°C.

Il est décrit des compositions comprenant de 5 à 90 % en poids de composé(s) (A), de 5 à 40 % en poids de composé(s) (B) et de 5 à 55 % en poids de composé(s) (C).

La présente invention concerne des compositions comprenant au moins un composé (A) choisi dans le groupe constitué des hydrofluorocarbures, des (hydro)fluorooléfines, (hydro)fluoroiodocarbures et des hydrocarbures, au moins un composé (B) choisi dans le groupe constitué des gaz rares, de l'azote, du dioxyde d'azote, du sulfure d'hydrogène et du dioxyde de carbone et au moins un composé organique fonctionnalisé (C) ayant une température d'ébullition à pression atmosphérique supérieure à 60°C, une température de fusion à pression atmosphérique < -20°C et/ou un point éclair > 50°C et de préférence > 70°C ; caractérisées en ce qu'elles comprennent de 5 à 90 % en poids de composé(s) (A), de 5 à 40 % en poids de composé(s) (B) et de 5 à 55 % en poids de composé(s) (C).

Ces compositions comprennent avantageusement de 15 à 80 % en poids de composé(s) (A), de 10 à 40 % en poids de composé(s) (B) et de 10 à 45 % en poids de composé(s) (C).

Comme hydrofluorocarbures, on peut citer notamment le difluorométhane, le difluoroéthane, le 1,1,1,2 tetrafluoroéthane, le 1,1,2,2 tetrafluoroéthane, le pentafluoroéthane, le pentafluoropropane, l'heptafluoropropane et le pentafluorobutane. Le difluorométhane, le difluoroéthane et le 1,1,1,2 tetrafluoroéthane sont préférés.

Lorsque le 1,1,1,2 tetrafluoroéthane est présent dans la composition, elle représente de préférence au plus 10 % du poids total.

Comme (hydro)fluorooléfines, on peut citer notamment les (hydro)fluoroéthylènes, les (hydro)fluoropropylènes et les (hydro)fluorobutylènes. Les (hydro)fluoropropylènes sont préférés.

Comme (hydro)fluoropropylènes, on peut citer notamment le difluoropropylène (HFO-1252), le trifluoropropylène (HFO-1243), le tetrafluoropropylène (HFO-1234) et le pentafluoropropylène (HFO-1225). Les hydrofluoropropylènes préférés sont le 1,3,3,3-tetrafluoropropylène (HFO-1234ze), le 2,3,3,3-tetrafluoropropylène (HFO-1234yf) et le 1,2,3,3,3-pentafluoropropylène (HFO-1225ye) ainsi que chacun de ses stéréoisomères. Le perfluoropropylène peut également convenir. Le 2,3,3,3-tetrafluoropropylène (HFO-1234yf) est particulièrement préféré.

Comme (hydro)fluoroiodocarbures on peut citer le trifluoroiodométhane (CF₃I).

Le dioxyde de carbone est de préférence choisi comme composé (B).

Comme composé organique fonctionnalisé (C), on peut citer notamment l'éthylène glycol, le diéthylène glycol, le triéthylène glycol, le propylène glycol, le dipropylène glycol, l'hexylène glycol, le 1,3-butanediol, le diacétone alcool (C₆H₁₂O₂), le diéthyl carbonate, le propylène carbonate, le méthyl-4 dioxolane-1,3 one-2, la N,N-diéthylformamide, l'aniline, l'hydroxy-4 méthyl-4 pentanone, la 3-methoxy-3 méthyl-1 butanol, la méthyldiéthanolamine, le tripropylène glycol méthyl éther, le dipropylène glycol methyl éther, le dipropylène glycol n_propyl éther, le dipropylène glycol n-butyl éther, la méthyl-5 heptanone-3, la diméthyl sulfone, l'adipate de diméthyl ester, glutarate de diméthyle, succinate de diméthyle, le diisobutyladipate, le carbonate de glycérol, la γ-butyrolactone, 1,3-benzène diol, le triméthyl phosphate, le 1-éthylpyrrolidin-2-one, le méthyl-1-pyrrolidone-2, le dipropylèneglycol méthyl éther acetate, le dimethyl isosorbide, nonafluorobutyl éthyl éther (C₄F₉OC₂H₅), acétate de 2-éthylhexyle, éthyle benzoate, diacétate d'éthylèneglycol, malonate d'éthyle, sulfure de dibutyle, triacétate de glycérine, éthylène glycol monomethyl ether, diethylène glycol monomethyl ether, triethylène glycol monomethyl ether, éthylène glycol dimethyl ether, diethylène glycol dimethyl ether, triethylène glycol dimethyl ether, tetraethylene glycol dimethyl ether, diethylène glycol monoethyl ether, diethylène glycol diethyl ether, éthylène glycol monobutyl ether, diethylène glycol monobutyl ether, propylene glycol methyl ether acetate, propylene glycol methyl ether propionate, ethylene glycol butyl ether acetate, diethylene glycol butyl ether acetate, dipropylène glycol méthyl éther acétate, triethylène glycol monobutyl ether, et polyethylène glycol dimethyl ether.

Les composés (C) préférés sont : le carbonate de propylène, le dipropylène glycol n-butyl éther, le tripropylène glycol méthyl éther, le tetraethylene glycol dimethyl éther, diethylène glycol diethyl ether, triethylène glycol monomethyl ether, triethylène glycol monobutyl ether, dipropylène glycol méthyl éther acétate et diacétate d'éthylèneglycol, perfluoro-1,3-dimethylcyclohexane, perfluorooctane, perfluoro-1,3,5-trimethylcyclohexane, perfluorotributylamine.

Les composés (C) avantageusement préférés sont : dipropylène glycol n-butyl ether, 3-methoxy-3-methyl 1-butanol, dipropylène glycol methyl ether, dipropylène glycol propyl ether, dipropylène glycol methyl ether acetate, ethyl diglycol acétate, diethylene glycol butyl ether, ethylhexyl-2-acetate, tetraethylene glycol dimethyl ether, glycéryl triacetate, butoxy-2-ethoxy 2 ethyl acetate, diethyl malonate, diethylène glycol diethyl ether, dimethyl glutarate, ethyl benzoate, triethylène glycol monobutyl ether, butyl sulfide et nonafluorobutyl ethyl ether.

A titre d'exemple de compositions particulièrement préférées on peut citer notamment celles comprenant du
- difluoromethane, du dioxyde de carbone et du diacetate d'éthylène glycol
- difluoroethane, du dioxyde de carbone et du diacetate d'éthylène glycol
- du 2,3,3,3-tetrafluoropropylène (HFO-1234yf), du dioxyde de carbone et du diacetate d'éthylène glycol.

Les compositions selon la présente invention conviennent aux systèmes à compression, pour des applications de refroidissement (système frigorifique) ou de chauffage (pompe à chaleur) ou dans des machines inversables dites parfois réversibles, produisant du froid pour le rafraîchissement ou de la chaleur pour le chauffage.

Avec les compositions de la présente invention, la présence d'une phase liquide à l'intérieur des échangeurs, comprenant le composé (C), et d'une phase gaz favorise les phénomènes de dissolution et d'évaporation suivant les niveaux de pression. Dans le condenseur, avec l'augmentation de la pression, les composés les plus volatiles, tel que le dioxyde de carbone, se dissolvent dans la phase liquide et c'est la chaleur de dissolution qui va s'ajouter à la chaleur de condensation. Avec la chute de pression à l'évaporateur, les composés les plus volatiles se libèrent de la phase liquide et absorbent la chaleur du milieu extérieur. Cette chaleur absorbée va s'ajouter à la chaleur d'évaporation des composés (A) moins volatiles et augmente ainsi la puissance de refroidissement et les performances du système. Les compositions selon la présente invention présentent également, suivant la nature du composant (C), l'avantage de fonctionner avec une quantité réduite de lubrifiants ou en l'absence de lubrifiant lorsqu'elles sont mises en oeuvre dans des compresseurs.

La présente invention a également pour objet l'utilisation des compositions décrites ci-dessus comme fluide de transfert d'énergie et fluide frigorigène, en particulier dans la production d'air conditionné.

Les compositions décrites précédemment conviennent particulièrement comme fluide frigorigène dans la climatisation des véhicules et peuvent remplacer partiellement ou en totalité le 1,1,1,2 tetrafluoroéthane.

### PARTIE EXPERIMENTALE

### A) Absorption du CO₂

On introduit 64 g de dioxyde de carbone dans un autoclave de 320 ml, équipé d'un capteur de température, d'un capteur de pression et d'un agitateur magnétique. Puis on y introduit progressivement le composé organique fonctionnalisé à l'aide d'une pompe volumétrique. La température de l'autoclave est maintenue à 40°C, à l'aide d'un bain thermostaté, pendant toute la durée des essais.

La pression du mélange résultant est reportée dans le Tableau 1.

**Tableau 1**

| % du composé organique fonctionnalisé (vol/vol) | 0 | 7 | 15 | 22 | 30 | 37 |
|---|---|---|---|---|---|---|
| | Pression (bar) | | | | | |
| carbonate de glycérol | 73,38 | 74,76 | 76,31 | 77,87 | 79,47 | 81,38 |
| Dipropyleneglycol n-Butyl Ether | 72,6 | 67,7 | 63,25 | 59,49 | 56,32 | 53,15 |
| Dipropylene Glycol | 70,56 | 70,55 | 70,39 | 70,16 | 70,09 | 69,75 |
| 3-méthoxy-3 méthyl 1 butanol | 72,58 | 67,71 | 64,71 | 62,79 | 59,92 | 57,29 |
| Dipropylèneglycol Méthyl Ether | 73,35 | 67,15 | 62,74 | 59,06 | 55,95 | 53,26 |
| diPropylene Glycol propyl ether | 72,53 | 68 | 63,96 | 60,5 | 57,54 | 54,79 |
| TriPropylene Glycol Methyl ether | 75,35 | 70,71 | 66,95 | 63,87 | 61,23 | 59,4 |
| tributyl borate | 69,59 | 66,64 | 65,4 | 64,84 | 63 | 61,32 |
| Dipropylèneglycol Méthyl Ether acétate | 72,51 | 64,89 | 58,92 | 54,17 | 50,16 | 46,82 |
| Ethyl diglycol acétate | 72,04 | 65,79 | 60,77 | 56,3 | 52,28 | 49,24 |
| Diethylène glycol dibutyl ether | 71,61 | 66,96 | 64,86 | 62,19 | 59,03 | 56,41 |
| éthylhexyl-2-acétate | 72,72 | 66,36 | 61,49 | 57,51 | 54,24 | 51,36 |
| tétraéthylène glycol diméthyl éther | 71,79 | 65,64 | 62,66 | 58,17 | 54,32 | 50,99 |
| Ethylène glycol diacétate | 72,49 | 63,11 | 56,14 | 50,8 | 46,55 | 43,17 |
| Glycéryl Triacétate | 72,44 | 67,14 | 61,42 | 56,69 | 52,58 | 49,24 |
| Butoxy-2-éthoxy-2-éthyle acétate | 71,79 | 66,25 | 61,57 | 57,09 | 53,2 | 49,87 |
| Diethyle malonate | 69,88 | 62,16 | 56,46 | 51,46 | 47,52 | 44,28 |
| diéthylèneglycol diéthyléther | 71,82 | 64,57 | 58,47 | 53,65 | 49,53 | 46,03 |
| diméthyle glutarate | 63,49 | 55,22 | 49,13 | 44,43 | 40,75 | 37,81 |
| Ethyle benzoate | 73,45 | 68,49 | 64,69 | 61,36 | 58,49 | 56,08 |
| triéthylèneglycol monobutyl éther | 71,61 | 69,46 | 68,13 | 66,55 | 64,6 | 62,51 |
| butyl sulfide | 72,45 | 68,48 | 66,05 | 63,45 | 61,16 | 59,2 |
| nonafluorobutyl éthyl ether | 70,98 | 61,96 | 55,53 | 50,87 | 47,12 | 44,15 |

### B) Performances thermodynamiques

Le tableau 2 donne les performances d'une composition comprenant du dioxyde de carbone (R744), diacétate d'éthylène glycol (EGDA) et éventuellement du difluoromethane (R32). Les propriétés thermodynamiques utilisées pour calculer les performances peuvent être résumées comme suit :
Le modèle prédictif PSRK (T. Holderbaum et J. Gmehling, « A group contribution equation of state based on UNIFAC », Fluid Phase Equilibrium, 1991, 70,251-265) a été utilisé pour le calcul des équilibres « liquide-vapeur » selon une approche symétrique et basé sur les méthodes de contribution de groupe. Il est composé d'une équation d'état, d'une fonction alpha, d'une règle de mélange et d'un modèle de solution UNIFAC (A. Fredenslund, RL Jones et JM Prausnitz, « Group contribution Estimation of Activity Coefficients in non-ideal liquid mixtures », AIChE J, 1975, 21,1086-1099).

Les paramètres de chacun des constituants (dioxyde de carbone, du difluoromethane et du diacétate d'éthylène glycol) proviennent de la base de donnée Dortmund Data Bank (DDB) (www.ddbst.de).

Nous avons étudié les systèmes binaires puis ternaires.

Les paramètres PSRK UNIFAC du binaire dioxyde de carbone et diacétate d'éthylène glycol sont disponibles dans la base de donnée Dortmund Data Bank (DDB) (www.ddbst.de).
S'agissant du système binaire difluoromethane et dioxyde de carbone, nous avons utilisé les données disponibles (F. Rivollet, A. Chapoy, C. Coquelet et D. Richon, « Fluid phase Equilibrium, 218, 2004, 95-101 » et R.A. Adams et F.P. Stein, « J Chem Eng Data, 16 (1971), 146-149 ».
Ne disposant d'aucune donnée dans la littérature, nous avons effectué des mesures pour le système binaire difluoromethane et diacétate d'éthylène glycol à l'aide de la technique synthétique à volume variable (M. Meskel-Lesavre, D. Richon et H. Renon, Ind. Eng. Chem. Fundam., 20,1981, 284-289).
En utilisant le modèle PSRK et les paramètres obtenus des systèmes binaires, nous avons prédit le point de bulle correspondant à trois mélanges ternaires (dioxyde de carbone, du difluoromethane et du diacétate d'éthylène glycol) et validé ces prédictions avec des valeurs mesurées utilisant la technique synthétique à volume variable.

Les équations d'état développées pour le mélange ternaire sont ensuite utilisées pour déterminer la courbe température-pression ainsi que les performances dans un cycle théorique de compression.

Les conditions sont :
- température extérieure = 35° C
- température intérieure = 5° C
- pas de perte de charge dans les échangeurs
- Compression isotropique
- Sous refroidissement 0°C à la sortie du condenseur
- Echangeur interne : sous refroidissement 25°C
- Titre vapeur à la sortie de l'évaporateur : 0,5 mole/mole

Pₑᵥₐₚ désigne la pression d'évaporation (kPa), P_{cond} désigne la pression de condensation (kPa), T_{sortie comp} désigne la température à la sortie du compresseur (°C), COP désigne le coefficient de performance et CAP désigne la capacité volumétrique (kJ/m3).

**Tableau 2**

| **R744 % en poids** | **EGDA % en poids** | | **P évap (kPa)** | **Pcond (kPa)** | **Tsortie comp (°C)** | **COP** | **CAP (kJ/m3)** |
|---|---|---|---|---|---|---|---|
| 60 | 40 | | 2449 | 5895 | 97 | 2,20 | 8197,24 |
| 50 | 50 | | 1908 | 5160 | 110 | 1,88 | 6327,27 |
| 40 | 60 | | 1288 | 4382 | 133 | 1,49 | 4257,64 |
| 30 | 70 | | 568 | 3529 | 202 | 0,92 | 1867,87 |
| | | | | | | | |

| **R744 % en poids** | **EGDA % en poids** | **R32 % en poids** | **P évap (kPa)** | **Pcond (kPa)** | **Tsortie comp (°C)** | **COP** | **CAP (kJ/m3)** |
|---|---|---|---|---|---|---|---|
| 35 | 35 | 30 | 1392 | 4094 | 112,9 | 2,23 | 5757,66 |
| 30 | 30 | 40 | 1293 | 3788 | 110 | 2,42 | 5699,30 |
| 25 | 25 | 50 | 1214 | 3498 | 106 | 2,64 | 5687,62 |
| 20 | 20 | 60 | 1147 | 3220 | 101 | 2,90 | 5700,29 |
| 40 | 27 | 33 | 1640 | 4477 | 105 | 2,45 | 6847,71 |
| 35 | 23 | 42 | 1513 | 4162 | 104 | 2,60 | 6686,71 |
| 30 | 20 | 50 | 1395 | 3854 | 102,9 | 2,73 | 6488,47 |
| 25 | 17 | 58 | 1293 | 3556 | 100 | 2,90 | 6292,26 |
| 20 | 13 | 67 | 1211 | 3275 | 97 | 3,12 | 6185,94 |
| 10 | 7 | 83 | 1058 | 2721 | 90 | 3,64 | 5888,68 |
| 40 | 13 | 47 | 1746 | 4509 | 97 | 2,85 | 7914,97 |
| 35 | 12 | 53 | 1602 | 4200 | 98 | 2,93 | 7504,62 |
| 30 | 10 | 60 | 1478 | 3900 | 97 | 3,03 | 7189,41 |
| 25 | 8 | 67 | 1366 | 3609 | 95 | 3,17 | 6894,49 |
| 20 | 7 | 73 | 1258 | 3318 | 94 | 3,29 | 6589,8 |

## Revendications

1. Compositions comprenant au moins un composé (A) choisi dans le groupe constitué des hydrofluorocarbures, des (hydro)fluorooléfines, (hydro)fluoroiodocarbures et des hydrocarbures, au moins un composé (B) choisi dans le groupe constitué des gaz rares, de l'azote, du dioxyde d'azote, du sulfure d'hydrogène et du dioxyde de carbone et au moins un composé organique fonctionnalisé (C) ayant une température d'ébullition à pression atmosphérique supérieure à 60°C, une température de fusion à pression atmosphérique < -20°C et/ou un point éclair > 50°C et de préférence > 70°C ; **caractérisées en ce qu'**elles comprennent de 5 à 90 % en poids de composé(s) (A), de 5 à 40 % en poids de composé(s) (B) et de 5 à 55 % en poids de composé(s) (C).

2. Compositions selon la revendication 1 **caractérisées en ce qu'**elles comprennent de 15 à 80 % en poids de composé(s) (A), de 10 à 40 % en poids de composé(s) (B) et de 10 à 45 % en poids de composé(s) (C).

3. Compositions selon l'une quelconque des revendications précédentes caractérisées en que le composé (B) est le dioxyde de carbone.

4. Compositions selon l'une quelconque des revendications précédentes **caractérisées en ce que** le composé (A) est choisi parmi le difluorométhane, le difluoroéthane et le 2,3,3,3 tetrafluoropropène.

5. Compositions selon l'une quelconque des revendications précédentes caractérisées en que le composé (C) est choisi parmi le carbonate de propylène, le dipropylène glycol n-butyl éther, le tripropylène glycol méthyle éther, le tetraethylene glycol dimethyl éther, diethylène glycol diethyl ether, triethylène glycol monomethyl ether, triethylène glycol monobutyl ether, dipropylène glycol méthyl ether acétate, ethylène glycol diacetate.

6. Utilisation des compositions selon l'une quelconque des revendications précédentes comme fluide frigorigène.

7. Utilisation des compositions selon l'une quelconque des revendications précédentes comme fluide de transfert d'énergie.

## Patentansprüche

1. Zusammensetzungen, umfassend mindestens eine Verbindung (A) aus der Gruppe bestehend aus wasserstoffhaltigen Fluorkohlenwasserstoffen, (Hydro)fluorolefinen, (wasserstoffhaltigen) Fluoriodkohlenwasserstoffen und Kohlenwasserstoffen, mindestens eine Verbindung (B) aus der Gruppe bestehend aus Edelgasen, Stickstoff, Stickstoffdioxid, Schwefelwasserstoff und Kohlendioxid und mindestens eine funktionalisierte organische Verbindung (C) mit einem Siedepunkt bei Normaldruck von mehr als 60 °C, einem Schmelzpunkt bei Normaldruck < -20 °C und/oder einem Flammpunkt > 50 °C und vorzugsweise > 70 °C; **dadurch gekennzeichnet, dass** sie 5 bis 90 Gew.-% Verbindung(en) (A), 5 bis 40 Gew.-% Verbindung(en) (B) und 5 bis 55 Gew.-% Verbindung (en) (C) umfassen.

2. Zusammensetzungen nach Anspruch 1, **dadurch gekennzeichnet, dass** sie 15 bis 80 Gew.-% Verbindung(en) (A), 10 bis 40 Gew.-% Verbindung(en) (B) und 10 bis 45 Gew.-% Verbindung(en) (C) umfassen.

3. Zusammensetzungen nach einem der vorhergehenden Ansprüche, **dadurch gekennzeichnet, dass** es sich bei der Verbindung (B) um Kohlendioxid handelt.

4. Zusammensetzungen nach einem der vorhergehenden Ansprüche, **dadurch gekennzeichnet, dass** die Verbindung (A) aus Difluormethan, Difluorethan und 2,3,3,3-Tetrafluorpropen ausgewählt ist.

5. Zusammensetzungen nach einem der vorhergehenden Ansprüche, **dadurch gekennzeichnet, dass** die Verbindung (C) aus Propylencarbonat, Dipropylenglykol-n-butylether, Tripropylenglykolmethylether, Tetraethylenglykoldimethylether, Diethylenglykoldiethylether, Triethylenglykolmonomethylether, Triethylenglykolmonobutylether, Dipropylenglykolmethyletheracetat und Ethylenglykoldiacetat ausgewählt ist.

6. Verwendung der Zusammensetzungen nach einem der vorhergehenden Ansprüche als Kältefluid.

7. Verwendung der Zusammensetzungen nach einem der vorhergehenden Ansprüche als Energieübertragungsfluid.

## Claims

1. Compositions comprising at least one compound (A) chosen from the group constituted by hydrofluorocarbons, (hydro)fluoroolefins, (hydro)fluoroiodocarbons and hydrocarbons, at least one compound (B) chosen from the group constituted by rare gases, nitrogen, nitrogen dioxide, hydrogen sulfide and carbon dioxide, and at least one functionalized organic compound (C) with a boiling point at atmospheric pressure of greater than 60°C, a melting point at atmospheric pressure < -20°C and/or a flash point > 50°C and preferably > 70°C; **characterized in that** they comprise from 5% to 90% by weight of compound (s) (A), from 5% to 40% by weight of compound(s) (B) and from 5% to 55% by weight of compound(s) (C).

2. Compositions according to Claim 1, **characterized in that** they comprise from 15% to 80% by weight of compound(s) (A), from 10% to 40% by weight of compound(s) (B) and from 10% to 45% by weight of compound(s) (C).

3. Compositions according to any one of the preceding claims, **characterized in that** compound (B) is carbon dioxide.

4. Compositions according to any one of the preceding claims, **characterized in that** compound (A) is chosen from difluoromethane, difluoroethane and 2,3,3,3-tetrafluoropropene.

5. Compositions according to any one of the preceding claims, **characterized in that** compound (C) is chosen from propylene carbonate, dipropylene glycol n-butyl ether, tripropylene glycol methyl ether, tetraethylene glycol dimethyl ether, diethylene glycol diethyl ether, triethylene glycol monomethyl ether, triethylene glycol monobutyl ether, dipropylene glycol methyl ether acetate and ethylene glycol diacetate.

6. Use of the compositions according to any one of the preceding claims, as a refrigerant fluid.

7. Use of the compositions according to any one of the preceding claims, as an energy-transfer fluid.
